# EUROPEAN PATENT SPECIFICATION

(11) **EP 2 998 312 B1**
(45) Date of publication and mention of the grant of the patent: **29.11.2017**
(21) Application number: 14798053.6
(22) Date of filing: 24.02.2014
(51) Int. Cl.: C07K 14/47, A61K 38/17, A61P 35/00

(54) **POLYPEPTIDE HAVING ANTI-TUMOR ACTIVITY AND USE THEREOF**
POLYPEPTID MIT ANTITUMORWIRKUNG UND VERWENDUNG DAVON
POLYPEPTIDE AYANT UNE ACTIVITÉ ANTI-TUMORALE ET SON UTILISATION

(30) Priority: 14.05.2013 CN 201310178099
(43) Date of publication of application: 23.03.2016
(73) Proprietor: Zhou, Jianwei, Nanjing, Jiangsu 211100 (CN)
(72) Inventor: ZHOU, Jianwei, Nanjing Jiangsu 211100 (CN); XU, Jin, Nanjing Jiangsu 211100 (CN); LI, Xuan, Nanjing Jiangsu 211100 (CN); LI, Aiping, Nanjing Jiangsu 211100 (CN)
(74) Representative: Hryszkiewicz, Danuta
(86) International application number: PCT/CN2014/072461
(87) International publication number: WO 2014/183491

(56) References cited:
- CN-A- 103 239 710
- US-B1- 6 808 893
- J BAI ET AL: "JWA regulates melanoma metastasis by integrin [alpha]V[beta]3 signaling", ONCOGENE, vol. 29, no. 8, 30 November 2009 (2009-11-30), pages 1227-1237, XP055289300, GB ISSN: 0950-9232, DOI: 10.1038/onc.2009.408

## Description

The invention relates to a series of anti-tumor polypeptides and a method for preparing an anti-tumor drug comprising the same.

The applicant is given to understand that cancer has become the second leading cause of death in the world. Malignant tumors are among the most dangerous diseases which greatly threaten human health. In China, the cancer - related mortality rate is 17.9%, which is the highest among all diseases, and the incidence rate thereof has an upward tendency. Seeking for effective anti-tumor drugs and methods to treat cancers is one of the most important research topics worldwide.

Tumor therapy accounts for comprehensive treatment, among which chemotherapy is one of the indispensable means. In recent years, with the significant development in anti-tumor drugs discovery, multiple anti-tumor drugs have emerged, thus have effectively prolonged the overall survival time of patients and improved their quality of life. Nevertheless, many problems have emerged at the same time, such as: drug resistance and side effects. Research has discovered that the therapeutic outcomes of chemical drugs and drug resistance development are both determined by the expression levels of certain genes. In other words, controlling the expression of certain genes in cells may help to inhibit the development of drug resistance, improve the therapeutic effects, reduce toxicity and side effects of drugs, improve quality of life, and eventually bring good news to more tumor patients.

JWA gene, also known as ARL6IP5 (GenBank: AF070523.1, 1998; LOCUS: AF070523, NM_006407), is an all-trans-retinoic acid (ATRA)-induciable gene, and firstly cloned from human primary tracheal-bronchial epithelial cells. JWA is a new cytoskeleton binding protein. During these years, the inventors of the present invention and his research team have been working on the relationship between the structure and function of JWA gene and the significance in cancer translational medicine. They achieved a series of meaningful findings: (1) JWA protein is a novel microtubule associated protein (MAPs) that can be bound to α-tubulin and β-tubulin subunits, and can response to environmental factors (e.g. cold shock, hot shock, taxinol, etc). In addition, both JWA and α-tubulin or β-tubulin subunits present synchronistic and co-localized distribution patterns in cells; (2) JWA protein regulates cellular mitosis and microtubule dynamic stability; (3) JWA protein suppresses tumor cell migration through mitogen-activated protein kinase (MAPK) signaling pathway as well as depolymerization and reorganization of microfilaments. Tumor cell migration is essential for tumor infiltration and metastasis, and high migrating ability of tumor cells increase tumor cell metastatic potential. The inventors of this patent have found that knocking down of JWA gene expression stably in human or mouse melanoma cells (A375, B16F10) can significantly increase their metastatic potential in mice. Moreover, melanoma cells with lower JWA expression present higher migration, invasion and adhesion capabilities *in vitro.* Further study has shown that JWA inhibited malignant tumor phenotypes, such as migration and metastasis by negatively regulating the expression of nuclear transcription factor Sp1, thus suppressing integrin αᵥβ₃ signaling pathway (J. Bai et al.: JWA regulates melanoma mestastasis by integrin alphavbeta3 signaling in Oncogene 29 (2010, 1227-1237, published on 30.11.2009).

As JWA inhibits tumor cell adhesion, infiltration, metastasis, and tumor angiogenesis, the mass production of JWA protein is imperative. However, at present, it is difficult to obtain purified JWA protein by using host expression system, thus making it infeasible to produce purified JWA protein by this means.

Moreover, the applicant understands that integrins are a class of molecules belonging to the glycoprotein receptor family, which are extensively present on cytomembrane surfaces and mediating adhesion between cells and the extracellular matrix (ECM). Integrin molecules are dimer molecules formed by non-covalent bonding of α and β subunits, and different combinations of the two subunits determine the specificity of protein-ligand, among which the function of integrin αᵥβ₃ is particularly important. High expression of integrin αᵥβ₃ is detected on the cell surface of many malignant tumors, while relatively low level or absent in normal tissues. In addition, integrin αᵥβ₃ can be specifically identified by arginine-glycine-aspartic acid (RGD) sequence in ligand molecules.

Many molecules exert their function in cytoplasm, which requires their targetting biomacromolecules to penetrate the cytomembranes. However, cytomembrane, the biological barrier, prevents many macromolecular drugs entering to cytoplasm, thus restricting the application of these materials in therapeutic field. Therefore, it is urgent needs to find out ways to deliver these biomacromolecules into cytoplasm. Traditional puncture is not recommended in drug delivery for their complex operation, inefficienty, cellular toxicity and immunogenicity. Cell-penetrating peptides (CPPs) are a class of short chain polypeptides capable of mediating endocytosis in a receptor-dependent or non-receptor-dependent manner. This characteristic makes them effectively transport biomacromolecules into cytoplasm, thereby providing a new and powerful tool for biotherapy. Cell-penetrating peptides (CPPs) are capable of mediating a series of bioactive molecules such as proteins, peptides, oligonucleotides, deoxyribonucleic acids (DNAs), plasmids, liposomes, etc *in vitro* or *in vivo* to enter various tissues and cells so as to exert their respective biological effects; and their cell membrane penetrating property not only is used in research of intracellular and extracellular transport of substances, but is also a potential effective biomolecular carrier to play a role in tumor treatment. Development of cell-penetrating peptide technology enables us to avoid potentially long-term impacts arising from the use of replicable cloned molecules, hence greatly accelerating the progress in research and development of small-molecule targeted drugs.

In view of the above-described problems, it is one objective of the invention to provide an anti-tumor polypeptide obtained from amino acid sequence of JWA protein through screening and identification. It is another objective of the invention to provide a method for preparing an anti-tumor drug comprising the same.

Inventors of this patent hold the view that artificial synthesis or expression of JWA protein by host expression system can hardly conduct investigation on the biological functions of JWA protein *in vivo* and *in vitro.* This could be due to the fact that the structure of JWA protein has three transmembrane domains or other unknown reasons. It is inferred by the inventors that the biological activity of JWA protein may be determined by a certain structural domain formed by several continuous amino acid residues in its primary structure, and the biological activity thereof may also be determined by several amino acid residues which are non-adjacent in its primary structure but adjacent in its tertiary structure. The inventors have continued the research in depth on this basis and eventually screened and identified a JWA functional anti-tumor polypeptide.

To achieve the above objective, in accordance with one embodiment of the invention, there is provided an anti-tumor polypeptide. An amino acid sequence of the polypeptide is represented by:
FPGSDRF;
X-FPGSDRF;
FPGSDRF-Z; or
X-FPGSDRF-Z;
where S is a phosphorylated serine residue; X and Z are an amino acid residue or an amino acid sequence, respectively;
X is one selected from the group consisting of F, (R)₉, (R)₉-F, 6-aminocaproic acid, 6-aminocaproic acid-F, 6-aminocaproic acid-(R)₉, and 6-aminocaproic acid-(R)g-F;
Z is one selected from the group consisting of A, (G)ₙ-RGD, and A-(G)ₙ-RGD; and n is an integer greater than or equal to 0.

In a class of this embodiment, a range of n is between 0 and 10.

In a class of this embodiment, an N-terminus of the polypeptide is acetylated and a C-terminus of the polypeptide is amidated.

In accordance with one embodiment of the invention, there is provided a method for preparing an anti-tumor drug comprising using the above anti-tumor polypeptide.

In a class of this embodiment, the tumor drug melanoma or gastric carcinoma.

Advantages of the anti-tumor polypeptide according to embodiments of the invention are summarized as follows: the polypeptide of the invention has a relatively short sequence and thus facilitates mass production. The polypeptide demonstrates significant anti-tumor activity at low dose, and when being used with chemicals (such as arsenic trioxide, i.e. As₂O₃, ATO), it can increase the toxicity of the chemicals to tumor cells. The polypeptide of the invention is not toxic to normal histocytes and has a vast application prospect

The invention is described herein below with reference to the accompanying drawings, in which:

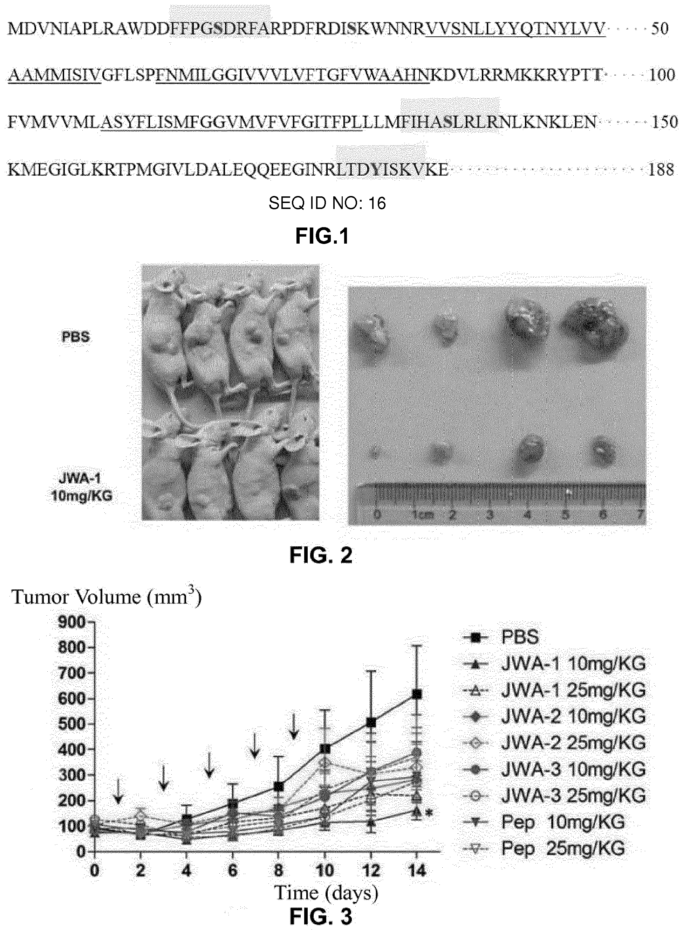

FIG. 1 is an amino acid sequence diagram of JWA protein. The underlined portions are transmembrane domains and the shaded portions are segments related to the embodiments,
   where the amino acids denoted by various letters are as follows: A: alanine; R: arginine; D: aspartic acid; Q: glutamine; E: glutamic acid; H: histidine; I: isoleucine; G: glycine; N: asparagine; L: leucine; K: lysine; M: methionine; F: phenylalanine; P: proline; S: serine; T: threonine; W: tryptophan; Y: tyrosine; V: valine;
FIG. 2 is a tumor formation comparison between the control group and the polypeptide JWA-1 (10 mg/kg) group in Example 2;
FIG. 3 is a graph of tumor volume changes in Example 2, where JWA1, JWA2, and JWA3 respectively denote three JWA single peptides, "pep" denotes an equivalent peptide blend of the three JWA single peptides, arrows denote drug administration times, and an asterisk * indicates *P*<0.05;
FIG. 4 is a tumor weight to body weight ratio graph of Example 2, where JWA1, JWA2, and JWA3 respectively denote three single JWA peptides, "pep" denotes an equivalent peptide blend of the three single JWA peptides, and an asterisk * indicates *P*<0.05;
FIG. 5 is a graph of mouse body weight changes in Example 2, where JWA1, JWA2, and JWA3 respectively denote three single JWA peptides, and "pep" denotes an equivalent peptide blend of the three single JWA peptides;
FIG. 6 is a tumor formation comparison among a control group, a non-pep group, and a polypeptide JWA-1 (10 mg/kg) group in Example 3;
FIG. 7 is a graph of tumor volume changes in Example 3, where arrows denote drug administration times;
FIG. 8 is a tumor weight to body weight ratio graph of Example 3;
FIG. 9 is a graph of mouse body weight changes in Example 3;
FIG. 10 is a tumor formation comparison among a non-pep group and a polypeptide JWA-1 (10 mg/kg) group, a polypeptide JWA-4 (10 mg/kg) group, a polypeptide JWA-5 (10 mg/kg) group, and a polypeptide JWA-6 (10 mg/kg) group, respectively in Example 4;
FIG. 11 is a graph of tumor volume changes in Example 4, where arrows denote drug administration times, and an asterisk * indicates *P*<0.05.
FIG. 12 is a tumor weight to body weight ratio graph of Example 4;
FIG. 13 is a graph of mouse body weight changes in Example 4;
FIG. 14 is a tumor formation comparison among a non-pep group, a polypeptide JWA-6 (50 mg/kg) group, a polypeptide JWA-6+RGD (10 mg/kg) group, and a polypeptide JWA-6+RGD (50 mg/kg) group in Example 5;
FIG. 15 is a graph of tumor volume changes in Example 5, where arrows denote drug administration times;
FIG. 16 is a tumor weight to body weight ratio graph of Example 5;
FIG. 17 is a graph of mouse body weight changes in Example 5;
FIG. 18 is a graph showing results of impact of various polypeptides on A375 cells after 24 hrs in Example 6;
FIG. 19 is a graph showing results of impact of various polypeptides on A375 cells after 48 hrs in Example 6.
FIG. 20 is a graph showing results of impact of various polypeptides on A375 cells after 72 hrs in Example 6;
FIG. 21 is a graph showing results of impact of various polypeptides on B16 cells after 24 hrs in Example 6;
FIG. 22 is a graph showing results of impact of various polypeptides on B16 cells after 48 hrs in Example 6;
FIG. 23 is a graph showing results of impact of various polypeptides on B16 cells after 72 hrs in Example 6.
FIG. 24 is a graph showing results of cell treatment using solely arsenic trioxide (ATO) after 24 hrs in Example 7, where asterisks ** indicate *P*<0.01;
FIG. 25 is a graph showing results of cell treatment using solely arsenic trioxide (ATO) after 48 hrs in Example 7, where asterisks ** indicate *P*<0.01;
FIG. 26 is a graph showing results of combined impact of arsenic trioxide (ATO) (0.5 µM, 1 µM, 2 µM, 5 µM) and polypeptide JWA-1 on A375 cells after 24 hrs in Example 7;
FIG. 27 is a graph showing results of combined impact of arsenic trioxide (ATO) (0.5 µM, 1 µM, 2 µM) and polypeptide JWA-1 on A375 cells after 48 hrs in Example 7;
FIG. 28 is a graph showing results of combined impact of arsenic trioxide (ATO) (1 µM, 2 µM, 5 µM) and polypeptide JWA-6 on A375 cells after 24 hrs in Example 7;
FIG. 29 is a graph showing results of combined impact of arsenic trioxide (ATO) (1 µM, 2 µM) and polypeptide JWA-6 on A375 cells after 48 hrs in Example 7;
FIG. 30 illustrates a Western Blot assay showing the expression of PARP-1 and caspase3 after treatment of A375 cells using solely polypeptide JWA-6 for 24 hrs and after treatment of A375 cells using polypeptide JWA-6 in combination with arsenic trioxide (ATO) for 24 hrs, respectively, in Example 7, where α-tubulin is an endogenous reference protein;
FIG. 31 illustrates a Western Blot assay showing changes in the expression of PARP-1 after treatment of B16 cells using solely polypeptide JWA-6 for 24 hrs and after treatment of B16 cells using polypeptide JWA-6 in combination with arsenic trioxide (ATO) for 24 hrs, respectively, in Example 7, where α-tubulin is an endogenous reference protein;
FIG. 32 is a graph of fluorescence intensity versus time after the entry of various cell-penetrating peptide (CPP) sequences and JWA-6 sequence with a concentration of 10µM into SGC7901 cells in Example 8;
FIG. 33 is a fluorescence intensity comparison between various cell-penetrating peptide (CPP) sequences and JWA-6 sequence in different doses after their effects on SGC7901 cells for 3 hrs and their entry thereinto in Example 8;
FIG. 34 is a fluorescence intensity comparison between various cell-penetrating peptide (CPP) sequences in different doses after their effects on BGC803 cells for 3 hrs and their entry thereinto in Example 8;
FIG. 35 is a fluorescence intensity comparison between various cell-penetrating peptide (CPP) sequences in different doses after their effects on HeLa cells for 3 hrs and their entry thereinto in Example 8;
FIG. 36 is a fluorescence intensity comparison between various cell-penetrating peptide (CPP) sequences in different doses after their effects on GES-1 cells for 3 hrs and their entry thereinto in Example 8;
FIG. 37 is a graph showing results of apoptosis detected by flow cytometry in Example 9;
FIG. 38 is a statistical histogram corresponding to FIG. 7;
FIG. 39 illustrates a Western Blot assay showing resultant expression levels of PARP-1 in Example 9;
FIG. 40 illustrates a Western Blot assay showing changes in apoptosis-related molecules in Example 9; and
FIG. 41 illustrates a Western Blot assay showing changes in PARP-1 molecules in Example 9.

For further illustrating the invention, experiments detailing an anti-tumor polypeptide and a method for preparing an anti-tumor drug comprising the same are described below. It should be noted that the following examples are intended to describe and not to limit the invention.

JWA protein contains 188 amino acids whose sequences are as shown in FIG. 1. The inventors of this patent have designed a series of random polypeptide sequence fragments directed at portions of JWA protein outside the transmembrance domains and carried out synthesis using existing method (such as fluorenylmethyloxycarbonyl (Fmoc) method), and thereafter performed screening and identification. The following embodiments provide detailed descriptions of screening and identification processes, and besides active polypeptides some representative non-active polypeptides were selected, although it should not be construed that only these polypeptides were involved in the research process.

### Example 1 Design and synthesis of polypeptides

The designed and synthesized polypeptides of this example are as shown in Table 1, where the amino acid sequences selected for JWA-1, JWA-2, and JWA-3 are consistent with the shaded portions in FIG. 1.

**Table 1 Designed and synthesized polypeptide sequences of Example 1**

| Name | Amino Acid Sequence (N-terminus - C-terminus) | Phosphorylation Site | SEQ ID NO. |
|---|---|---|---|
| JWA-1 | 6-aminocaproic acid-FFPGSDRFA | S phosphorylated | 1 |
| JWA-2 | 6-aminocaproic acid-FIHASLRLR | S phosphorylated | 2 |
| JWA-3 | 6-aminocaproic acid-LTDYISKV | Y phosphorylated | 3 |
| JWA-4 | 6-aminocaproic acid-GSD | S phosphorylated | 4 |
| JWA-5 | 6-aminocaproic acid-PGSDR | S phosphorylated | 5 |
| JWA-6 | 6-aminocaproic acid-FPGSDRF | S phosphorylated | 6 |
| JWA-7 | 6-aminocaproic acid-FFPGSDRFA | Not phosphorylated | 7 |

During synthesis of the aforementioned polypeptide sequences, the N-terminus thereof may be acetylated and the C-terminus thereof amidated to prevent rapid degradation of the polypeptides.

### Example 2 Selecting polypeptides having anti-tumor activity from JWA-1, JWA-2, and JWA-3

Human melanoma A375 cells in the logarithmic phase of growth were taken and placed under sterile conditions to prepare a 5×10⁶/200 µL cell suspension, and 200 µL cell suspension was separately inoculated under the dorsal skin of various BALB/c nude mice. An electronic digital vernier caliper was used to measure the long-axis and short-axis diameters of the transplanted tumors, and the formula: Tumor Volume (TV) = 1/2 × Long-axis Diameter × Short-axis Diameter² was used to calculate the sizes of the tumors. The mice were randomly grouped after the tumors grew to a volume of about 100 mm³.

Polypeptides JWA-1, JWA-2, JWA-3, and an equivalent peptide blend (Pep) of the three polypeptides were injected into the tumors of the various groups of mice respectively, and the doses used were 25 mg/kg and 10 mg/kg respectively; the negative control group was injected with an equal amount of phosphate-buffered saline (PBS) sterile solution. The polypeptide injection groups and the control group were administered on alternate days (48 hrs interval) by injection for 5 times; and the body weight of the mice and their tumor diameters were measured at each administration. After completion of the experiment the mice were put to death, tumors were separated from the bodies and the tumor weights measured.

The results were recorded as shown in FIGS. 2-5. In comparison with the control group (PBS group), the inhibition effect on subcutaneous tumor growth in nude mice in the 10 mg/kg polypeptide JWA-1 treated group was more significant, and the tumor weight to body weight ratio thereof was also smaller (P<0.05).

In addition, there was no statistical difference in average body weight of mice between the groups, and no drug toxicity reaction in the mice was observed during the experiment.

Melanoma is a highly malignant tumor, and the results of this example indicated that intratumoral injection of polypeptide JWA-1 in a dose of 10 mg/kg can significantly inhibit xenograft tumor growth of human melanoma A375 cells in nude mice.

### Example 3 Selecting the polypeptide JWA-1 dose having the optimal anti-tumor effect, and probing the impact of polypeptide phosphorylation on anti-tumor effect

The objective of this example was to ascertain the dose-effect relation between JWA polypeptides and tumor inhibition.

In this example, the method for dorsal subcutaneous tumor bearing of BALB/c nude mice by inoculation of A375 cells is the same as Example 2.

The aforementioned BALB/c nude mice inoculated with human melanoma A375 cells by intratumoral injection were separately treated using polypeptide JWA-1 of low dose (5 mg/kg), medium dose (10 mg/kg) and high dose (15 mg/kg); at the same time, a sterile PBS negative control group and a 10 mg/kg non-pep injection group (referred to simply as "non-pep group", the polypeptide sequence used was SEQ ID NO. 15; 6-aminoacetic acid-EEMQRR, the N-terminus thereof being acetylated and the C-terminus thereof amidated) were established. The establishment of the non-pep group was for observing whether JWA polypeptides have any specific anti-tumor effect.

To verify the impact of phosphorylation of S in polypeptide JWA-1 on the anti-tumor effect thereof, a non-phosphorylated polypeptide JWA-7 injection group (10 mg/kg) was added in the aforementioned experiment.

The various groups were administered by intratumoral injection on alternate days for 5 times consecutively; and the body weight of the mice and their tumor diameters were measured at each administration. After completion of the experiment the mice were put to death, tumors were separated from the bodies and the tumor weights measured.

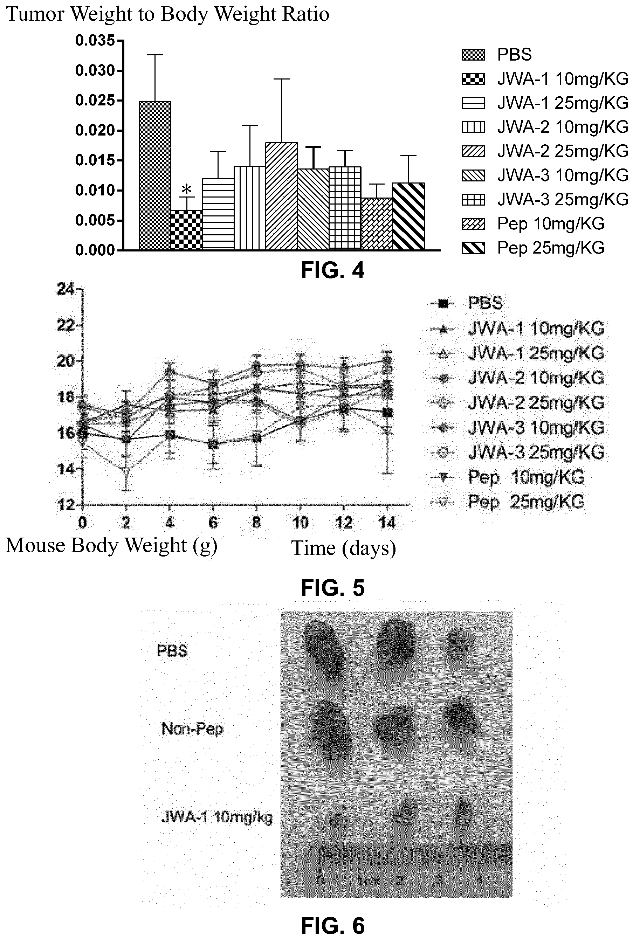

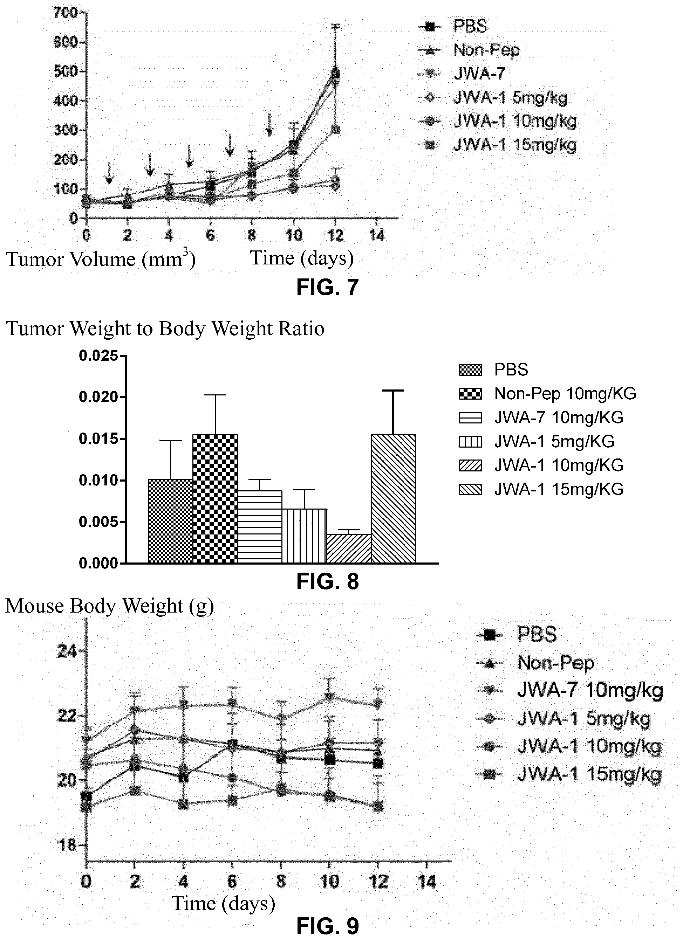

The results were recorded as shown in FIGS. 6-9. The 10 mg/kg polypeptide injection group demonstrated significant tumor inhibition effect, and no trend of enhanced effect was detected along with dose increase, with the effect on the 5 mg/kg JWA-1 injection group similar to that on the 10 mg/kg group. Although the reason for non-dependence on dose has not been ascertained, the ability of JWA-1 to inhibit the growth of transplanted tumors in nude mice has been verified.

At the same time, the non-peptide (non-pep) group and the polypeptide JWA-7 injection group demonstrated no tumor inhibition effect. It can be known from the results of the polypeptide JWA-7 injection group that the phosphorylation of S in polypeptide JWA-1 is an imperative condition for its anti-tumor effect; hence it is not necessary to consider the effect of non-phosphorylated polypeptides.

In addition, there was no statistical difference in average body weight of mice between the groups, and no toxicity reaction in the mice was observed during the experiment.

### Example 4 Selecting the shortest functional unit of polypeptide JWA-1

The objective of this example was to explore whether polypeptide JWA-1 still has anti-tumor effect after its length reduction.

To this end, polypeptides JWA-4, JWA-5, and JWA-6 were used in this example, and these polypeptides are based on polypeptide JWA-1 with 3, 2, or 1 amino acid(s) reduced from the N-terminus and C-terminus respectively.

In this example, the method for dorsal subcutaneous tumor bearing of BALB/c nude mice by inoculation of A375 cells is the same as Example 2.

The BALB/c nude mice inoculated with human melanoma A375 cells were separately treated using 10 mg/kg polypeptides JWA-1, JWA-4, JWA-5, and JWA-6; at the same time, a 10 mg/kg non-pep injection group (referred to simply as "non-pep group", the polypeptide sequence used was the same as Example 3) was established.

The various groups were administered by intratumoral injection on alternate days for 5 times consecutively; and the body weight of the mice and their tumor diameters were measured at each administration. After completion of the experiment the mice were put to death, tumors were separated from the bodies and the tumor weights measured.

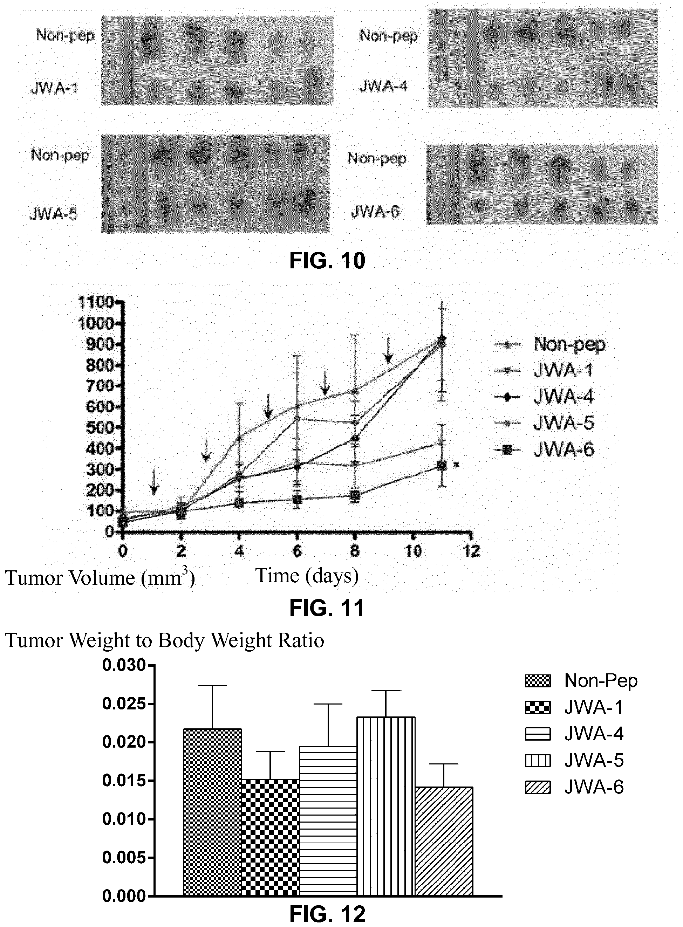

The results were recorded as shown in FIGS. 10-13. Polypeptide JWA-6 demonstrated similar and even better tumor inhibition effect compared to polypeptide JWA-1, whereas polypeptides JWA-4 and JWA-5 demonstrated significantly lower effect than polypeptide JWA-6. It can therefore be known from the results that polypeptide JWA-6 is the shortest functional unit of polypeptide JWA-1.

In addition, there was no statistical difference in average body weight of mice between the groups, and no toxicity reaction in the mice was observed during the experiment.

### Example 5 Selecting a functional polypeptide having polypeptide JWA-6 as the active site and specifically identifying Integrin αᵥβ₃

The tumor inhibition effects of JWA functional polypeptides observed in Examples 2-4 were all results of direct intratumoral injection of the polypeptides. Taking into consideration that direct intratumoral injection can be difficult during clinical application, intraperitoneal injection was used to observe whether the JWA functional polypeptide still has good anti-tumor effect.

It can be known from prior art that the expression of integrin αᵥβ₃ is increased on the cell surfaces of many malignant tumors including melanoma, and that arginine-glycine-aspartic acid (RGD) sequence can specifically identify integrin αᵥβ₃. Therefore, this example further selected a functional polypeptide from among intraperitoneally injected polypeptides based on polypeptide JWA-6 and connected with an arginine-glycine-aspartic acid (RGD) sequence.

The polypeptide designed and synthesized in this example was JWA-6+RGD whose sequence was SEQ ID NO. 8, i.e. 6-aminocaproic acid-FPGSDRF-GGGG-RGD, where amino acid S was phosphorylated. In addition, the N-terminus of the polypeptide sequence can be acetylated and the C-terminus thereof amidated during synthesis to prevent rapid degradation of the polypeptide.

In this example, non-obese diabetic/severe combined immunodeficiency (NOD/SCID) mice were chosen for tumor-bearing model and the method for dorsal subcutaneous tumor bearing by inoculation of human melanoma A375 cells is the same as Example 2. The mice were randomly divided into four treatment groups after the tumors grew to a volume of about 100 mm³.

Taking into consideration the polypeptide dose reaching the tumors partially after intratumoral injection, on the basis of the 10 mg/kg polypeptide used in the preceding example, a 50 mg/kg polypeptide group was added in this example, a single JWA-6 polypeptide at a dose of 50 mg/kg was used as a control group for reviewing the targeted anti-tumor effect, and a 50 mg/kg non-pep group was used to compare the specific anti-tumor effect of the JWA functional polypeptide.

Then, 50 mg/kg polypeptide JWA-6, 10 mg/kg polypeptide JWA-6+RGD, 50 mg/kg polypeptide JWA-6+RGD, and 50 mg/kg non-pep (the polypeptide sequence used was the same as Example 3) were intraperitoneally injected. The various groups were administered with injection on alternate days for 5 times consecutively; and the body weight of the mice and their tumor diameters were measured at each administration. After completion of the experiment the mice were put to death, tumors were separated from the bodies and the tumor weights measured.

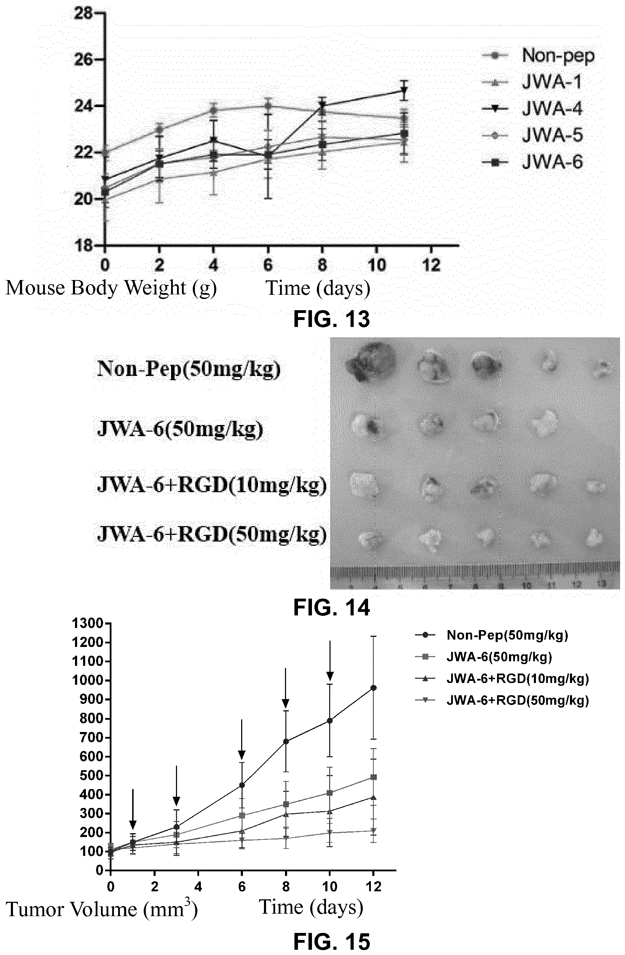

The results were recorded as shown in FIGS. 14-17. When administered by intraperitoneal injection, the 50 mg/kg polypeptide JWA-6 and 10 mg/kg polypeptide JWA-6+RGD both demonstrated a certain degree of tumor inhibition effect, while the 50 mg/kg polypeptide JWA-6+RGD demonstrated very obvious tumor inhibition effect.

In addition, there was no statistical difference in average body weight of mice between the groups, and no toxicity reaction to these three polypeptides in the mice was observed during the experiment.

In this example, 4 G (glycine) molecules were added between JWA-6 and RGD sequence, the purpose of which was to allow the JWA-6 polypeptides having anti-tumor activity to have a free space after the binding of RGD to the tumor cell surfaces so as to enhance the biological effects of the JWA-6 polypeptides. The inventors of the invention discovered through research that the number of G (glycine) molecules inserted between JWA-6 and RGD sequence may vary and may be greater than or equal to zero, but preferably smaller than or equal to ten.

Firstly, the following polypeptides containing RGD sequence were designed and synthesized (see Table 2).

**Table 2 Polypeptides containing RGD sequence**

| Name | Amino Acid Sequence (N-terminus - C-terminus) | Phosphorylation Site |
|---|---|---|
| JWA-6+RGD0 | 6-aminocaproic acid-FPGSDRF-RGD | S phosphorylated |
| JWA-6+RGD1 | 6-aminocaproic acid-FPGSDRF-G-RGD | S phosphorylated |
| JWA-6+RGD3 | 6-aminocaproic acid-FPGSDRF-GGG-RGD | S phosphorylated |
| JWA-6+RGD7 | 6-aminocaproic acid-FPGSDRF-(G)₇-RGD | S phosphorylated |
| JWA-6+RGD10 | 6-aminocaproic acid-FPGSDRF-(G)₁₀-RGD | S phosphorylated |

During synthesis of the aforementioned polypeptide sequences, the N-terminus thereof may be acetylated and the C-terminus thereof may be amidated to prevent rapid degradation of the polypeptides.

Thereafter, the tumor bearing mice were obtained using the foregoing method of this example and divided into seven injection groups which were intraperitoneally injected with polypeptides JWA-6+RGD, JWA-6+RGD0, JWA-6+RGD1, JWA-6+RGD3, JWA-6+RGD7, JWA-6+RGD10, and non-pep (the polypeptide sequence used was the same as Example 3) at a dose of 50 mg/kg. The various groups were administered by intraperitoneal injection on alternate days for 5 times consecutively; and the body weight of the mice and their tumor diameters were measured at each administration. The day of first administration was taken as the first day, and the experiment was stopped on the twelfth day when the mice were put to death, and tumors were separated from the bodies and the tumor weights measured.

Because of space constraints, the actual experimental data is not listed herein. The experimental data indicated that the anti-tumor activity of the polypeptides will not be affected when the number of glycine molecules between RGD sequence and JWA-6 is in the range of 0-10.

### Example 6 In vitro effects of polypeptides JWA-1, JWA-5, and JWA-6 on the activity of human melanoma A375 cells and mouse melanoma B16F10 cells

The objective of this example was to explore the in vitro effects of JWA functional polypeptides on the activity of melanoma cells.

B16-F10 or A375 cells in the logarithmic phase of growth were taken and inoculated evenly on a 96-well plate to a density of 5×10³ cells/well, and after 24 hrs of cell attachment different doses (0 µM, 5 µM, 10 µM, 25 µM, 50 µM) of polypeptide JWA-1, JWA-5, JWA-6 or non-pep (the polypeptide sequence used was the same as Example 3) were used to treat the cells for 24 hrs, 48 hrs and 72 hrs respectively. When the time for observation was over, the original culture medium was removed from the 96 wells and each well was added with a liquid mixture of CCK-8 reagent and new culture medium at a ratio of 1:10 was added to each well, and 2 hrs thereafter absorbance at 450nm was read and converted to a percentage relative to the untreated group. Four duplicate samples were taken from each group and tested thrice.

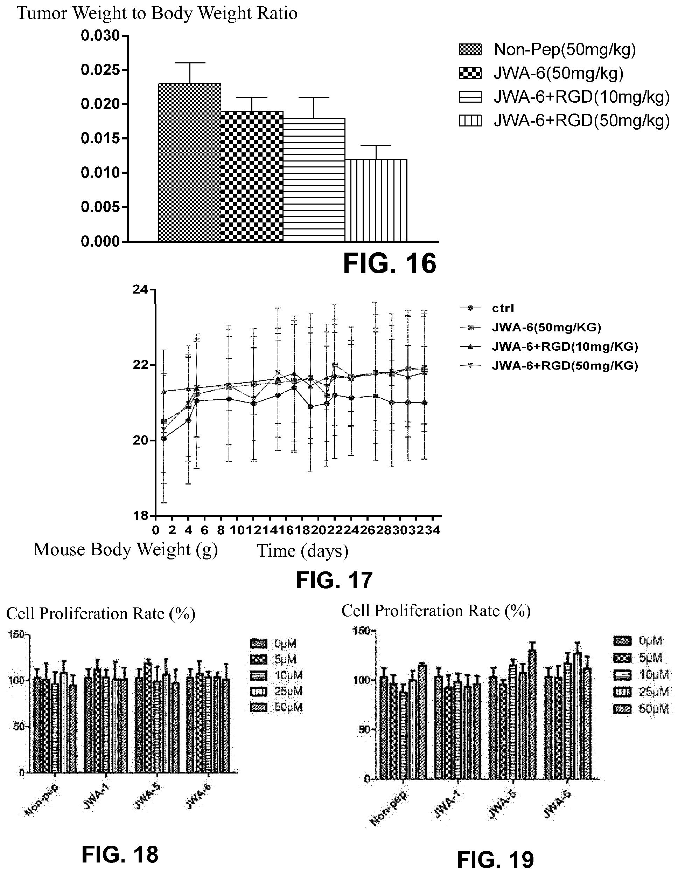

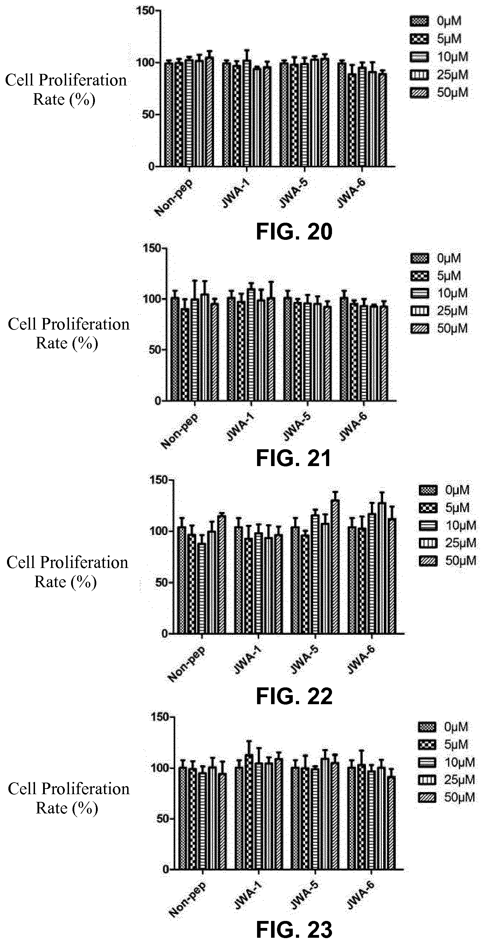

The results were recorded as shown in FIGS. 18-23. In comparison with the control group, pure polypeptides JWA-1, JWA-5, and JWA-6 were found to have no impact on the activity of A375 cells and B16-F10 cells after acting thereon extracorporeally.

### Example 7 Combined effect of polypeptides JWA-1, JWA-6, and As₂O₃ (ATO) on apoptosis of human melanoma A375 cells

The results of Example 6 indicated that treatment of human melanoma A375 cells with pure JWA functional polypeptides generated no cytotoxicity. Taking into consideration that in vivo experiments are far more complex than in vitro experiments, the research team led by the inventors of the invention discovered in preliminary study that JWA gene plays an important role in regulating ATO induced apoptosis, highly expressed JWA gene significantly intensifies ATO-induced tumor apoptosis, and mitogen-activated protein kinase (MAPK) signaling pathway takes part in the process. Therefore, the objective of this example was to determine whether the combined use of JWA functional polypeptides and ATO can enhance ATO induced apoptosis of melanoma cells.

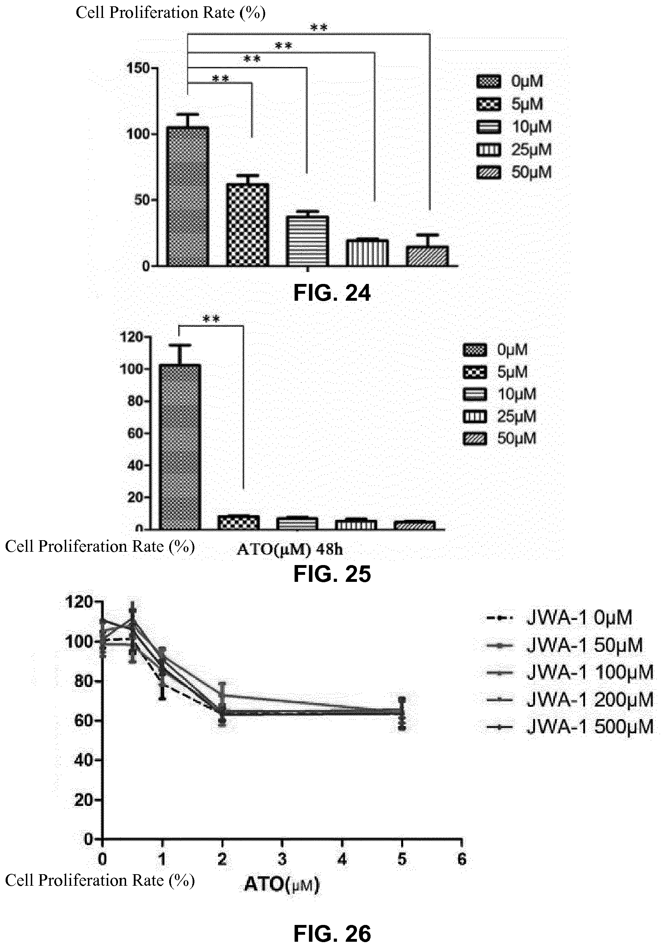

Firstly, the dose and time for treatment of A375 cells with ATO were determined. A375 cells in the logarithmic phase of growth were conventionally digested and inoculated evenly on a 96-well plate to a density of 5×10³ cells/well, and after 24 hrs of cell attachment different doses (5 µM, 10 µM, 25 µM, 50 µM) of ATO were used to treat the cells for 24 hrs and 48 hrs respectively. It was discovered that the activity of A375 cells was reduced by 40% after 24 hrs of treatment with 5 µM ATO (as shown in FIG. 24), and that the cell growth inhibition rate was approximately 100% after 48 hrs of treatment with different doses of ATO (as shown in FIG. 25). Therefore, ATO with a concentration smaller than or equal to 5 µM was chosen to treat the cells for 24 hrs for subsequent reference of dose and time for combined medication.

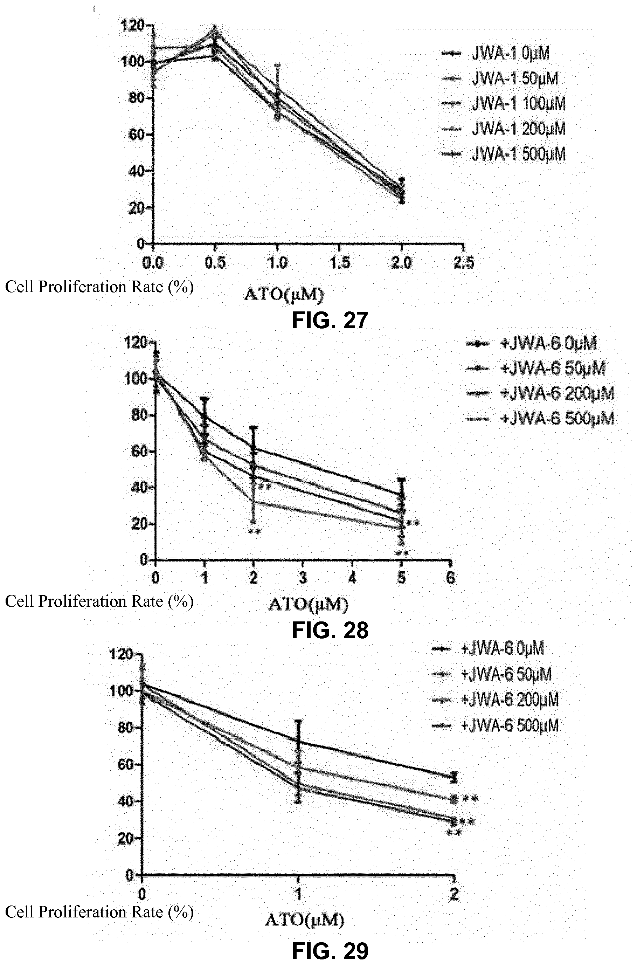

After determining the time and dose of ATO acting on the cells, polypeptides JWA-1 and JWA-6 at a dose of 50 µM, 100 µM, 200 µM and 500 µM respectively were used in combination with ATO at a dose of 0.5 µM, 1 µM, 2 µM and 5 µM to treat A375 cells for 24 hrs and 48 hrs respectively, after which the cytoactivity was examined and JWA-1 was found to have no synergistic effect with ATO while the combined used of JWA-6 and ATO exerts obvious toxicity to A375 cells (as shown in FIGS. 28-29).

The combined effect of ATO and polypeptide JWA-6 on the self-destruction of protein expression was also examined. A375 cells in the logarithmic phase of growth were conventionally digested and 5×10⁵ cells were inoculated evenly in a 60 mm cell culture dish, and different doses of ATO were used to individually treat the cells for 24 hrs, or different doses of ATO in combination with different doses of polypeptide JWA-6 were used to treat the cells for 24 hrs, after which 0.18 mL of RIPA lysis buffer containing 0.5% phenylmethanesulfonyl fluoride (PMSF) was added to extract protein. After centrifugation at 12000 g for 15 minutes, the supernatant was taken, protein concentration was examined and the protein was boiled. Based on the molecular weight measured, a polyacrylamide gel having a suitable concentration was chosen for protein electrophoresis where each well was added with 70 µg of protein, and the conditions for electrolysis were 60 V, 30 minutes and 90 V, 1-1.5 hrs. After completion of the electrolysis, semi-dry transfer was performed to transfer the protein from the gel onto a polyvinylidene difluoride (PVDF) membrane. After completion of the transfer, the membrane was blocked with 5% skim milk at room temperature for 1-2 hrs, after which membrane cleaning was performed 3 times (each lasting 5 minutes) using Tris-buffered Saline with Tween (TBST) containing 0.1% Tween-20, and incubation of the corresponding antibody was performed overnight at 4°C. On the next day, membrane cleaning was performed 3 times (each lasting 5 minutes) using Tris-buffered Saline with Tween (TBST) containing 0.1% Tween-20; incubation of secondary antibody was performed at room temperature for 1-2 hrs; and again membrane cleaning was performed 8 times (each lasting 5 minutes) using Tris-buffered Saline with Tween (TBST) containing 0.1% Tween-20. The membrane was added with enhanced chemiluminescence (ECL) liquid and exposed.

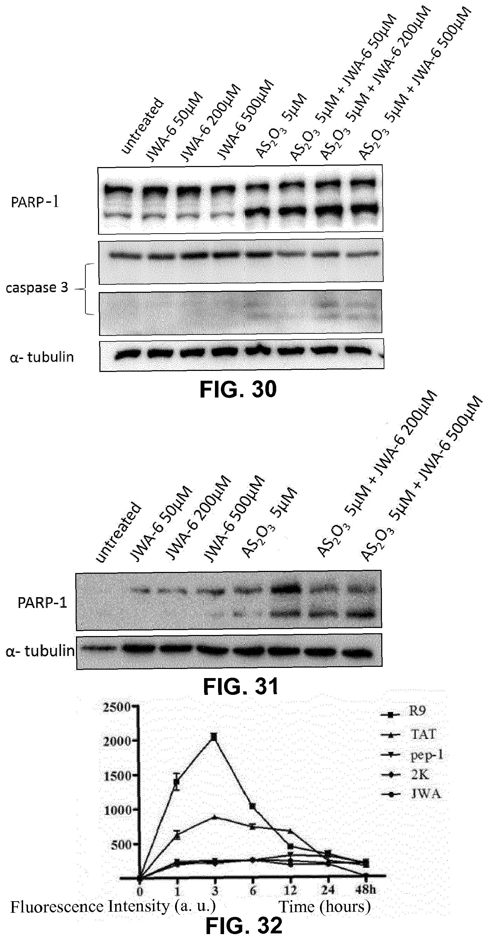

Through Western Blot experiment, it was discovered that compared to the untreated group and the group singly using ATO, caspase-3 spliceosome and PARP-1 spliceosome in the combined medication group of ATO and polypeptide JWA-6 were significantly increased, which indicated that the combined action of ATO and polypeptide JWA-6 further activated the mechanism of apoptosis to accelerate the apoptosis of A375 cells (as shown in FIG. 30). In addition, changes of PARP-1 were also seen in B16 cells (as shown in FIG. 31).

### Example 8 Selecting cell-penetrating peptides (CPPs) capable of bringing JWA functional polypeptides into cells

To enable JWA functional polypeptides to effectively penetrate into tumor cells, four typical CPPs: R9, TAT, pep-1, 2K (see Table 3), and a blank control having only JWA-1 were selected, the cell-penetrating peptide (CPP) with the highest cell-penetrating effect, its optimal action concentration and optimal action time were selected from the cell model.

**Table 3 Cell-penetrating peptide (CPP) sequences**

| Name | Amino Acid Sequence (N-terminus - C-terminus) | SEQ ID NO. |
|---|---|---|
| R9 | 6-aminocaproic acid-RRRRRRRRR | 9 |
| TAT | 6-aminocaproic acid-RKKRRQRRR | 10 |
| pep-1 | 6-aminocaproic acid-KETWWETWWTEWSQPKKKRKV | 11 |
| 2K | 6-aminocaproic acid-YGRKKRRQRRR | 12 |

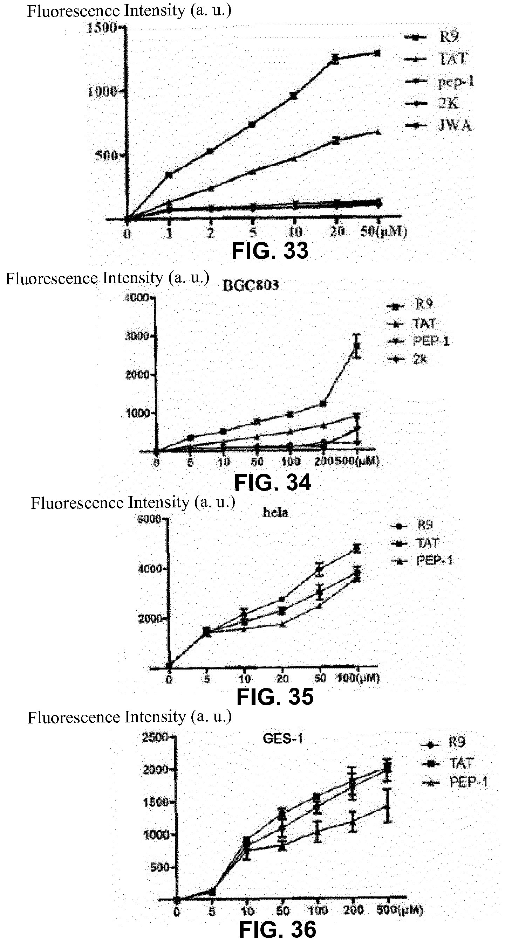

The fluorescence was at the highest level when cell-penetrating peptides (CPPs) were found to have entered the cells for 3 hrs on SGC7901 gastric carcinoma model; and when the concentration was 20 µM the fluorescence was balanced, and the differences between R9 group and other groups were the most significant (as shown in FIGS. 32-33). In addition, the same effects were proven in BGC803 cells, HeLa cells and normal gastric epithelial cells GES-1 (as shown in FIGS. 34-36).

Therefore, R9 having a relatively better effect was chosen in subsequent experiments as the cell-penetrating peptide (CPP) bringing JWA functional polypeptides into cells to exert their biological function.

To facilitate the use of fluorescence microscopy to directly observe whether polypeptides have the ability to enter cells, the N-terminuses of the aforementioned sequences and JWA-1 were modified with fluorescein isothiocyanate (FITC) and the C-terminuses thereof were amidated.

### Example 9 Combined effect of JWA functional polypeptides having cell-penetrating peptide (CPP) sequences and As₂O₃ (ATO) on tumor apoptosis

The objective of this example was to explore whether JWA functional polypeptides having CPP sequences can directly induce tumor apoptosis or accelerate drug-induced tumor apoptosis.

Six groups with different treatment methods directed against SGC7901 cells were designed: blank control group; transfection Flag-control group; transfection Flag-JWA group; transfection Flag-JWA and ATO combined treatment group; ATO treatment group; and polypeptide CPP-1 and ATO combined treatment group. After 24 hrs of treatment for each group, Annexin V-PE/PI flow cytometry was used to examine apoptosis and Western Blot was used to detect the level of PARP-1.

Flag-JWA was recombinant expression plasmid with JWA gene; Flag-control was used as blank plasmid control; the sequence of polypeptide CPP-1 was SEQ ID NO. 13, i.e. 6-aminocaproic acid-(R)₉-FFPGSDRFA, and the amino acid S was phosphorylated.

Cell transfection group: liposome mediated transfection (Lipofectamine™ 2000 Transfection Kit) was used. SGC7901 cells were trypsinized, diluted with antibiotic-free DMEM medium containing 10% fetal bovine serum (FBS), rapidly homogenized and added into various wells of a 6-well plate; transfection was performed within 24 hrs at a dose of 4 µg/well for Flag-control and 4 µg/well for Flag-JWA 4 µg/well; 250 µL of preheated serum-free and antibiotic-free DMEM medium was added; liposome was added to the 250 µL of serum-free and antibiotic-free DMEM medium at a ratio of 10 µL/well; after incubation at room temperature for 5 minutes, the two tubes were thoroughly mixed and placed at room temperature for 20 minutes, during which the culture medium in the dish was removed and replaced with 2 mL of serum-supplemented antibiotic-free DMEM medium. 500 µL of the mixture was added into various dishes, and the serum-supplemented antibiotic-free DMEM medium was replaced after 5 hrs to continue the cultivation for 24 hrs.

Transfection plasmid plus ATO treatment group: the method of transfection was the same as mentioned above. The serum-supplemented antibiotic-free DMEM medium containing 5 µM ATO was replaced after 5 hrs to continue the cultivation for 24 hrs.

Polypeptide CPP-1 plus ATO treatment group: SGC7901 cells were trypsinized, diluted with a DMEM complete culture medium, rapidly homogenized and added into 35mm dishes; the trypsinized cell culture was placed in a 5% CO₂ incubator for incubation at a temperature of 37°C to reach a degree of polymerization of 80%. 20µM polypeptide JWA-1-CPP working medium and 5µM ATO working medium were prepared. Polypeptide CPP-1 was first used to treat the cells for 3 hrs, and it was replaced with ATO working medium to treat the cells for 24 hrs.

In normal cells, phosphatidyl serine (PS) are distributed on the inside of cell membrane lipid bilayer, and at the early stage of apoptosis the phosphatidyl serine (PS) in cell membranes is turned over from the inside of lipid membrane to the outside thereof. Annexin V is a Ca²⁺-dependent phospholipids-binding protein with a molecular weight of between 35 and 36 kD and a high affinity for phosphatidyl serine (PS), therefore it can be bound with membranes of cells at early stage of apoptosis through phosphatidyl serine (PS) with exposed cellular lateralis. Annexin V was labeled with fluorescein phycoerythrin (PE); fluorescence microscopy or flow cytometry could be employed to examine apoptosis with the labeled Annexin V used as a fluorescent probe. 7-AAD (7-amino-actinomycin D) is a nucleic acid dye that cannot pass through normal plasmalemmas. Along with the progress of cell apoptosis, the permeability of plasmalemmas to 7-AAD is gradually increased and coupled with the controlled degradation of DNA during apoptosis, 7-AAD can emit bright red fluorescence under excitation by excitation light with suitable wavelength. Cells are divided into three group by the fluorescence intensity of 7-AAD labeling DNA: strong fluorescence of 7-AAD indicates dead cells, weak fluorescence of 7-AAD indicates apoptatic cells, and undetectable fluorescence of 7-AAD indicates normal active cells.

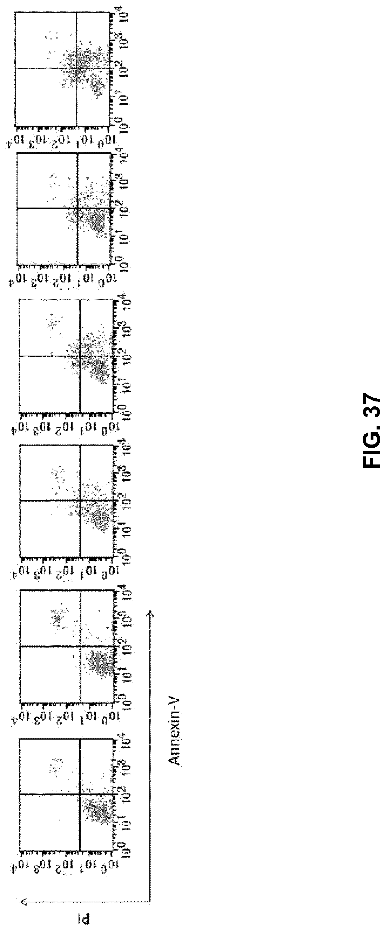

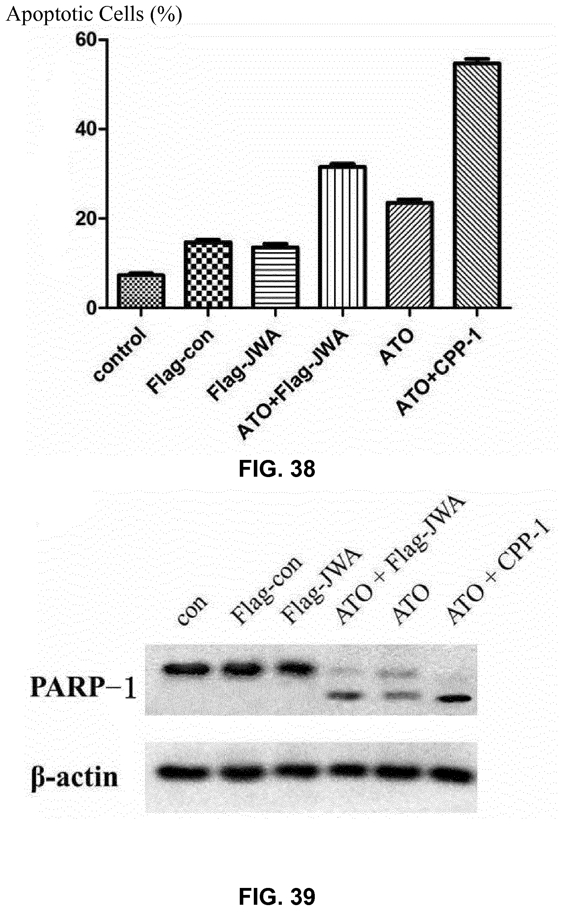

After treatment of the various treatment groups, the cells were trypsinized to prepare a cell suspension, Annexin V-PE/7-AAD staining was performed, and flow cytometry was used to examine apoptosis; the results were recorded as shown in FIG. 37. The foregoing results were plotted using Graphpad prism 5 to obtain a histogram (as shown in FIG. 38). The results indicated that: there was no statistical difference in apoptasis rate between the transfection Flag-JWA group and the transfection Flag-control group; the apoptasis rate of the polypeptide CPP-1 and ATO combined action group was the highest at 55.76%, which was higher than that of the transfection Flag-JWA and ATO combined action group (32.87%) and higher than that of the pure ATO treatment group (24.69%).

SGC7901 cells were treated using the foregoing treatment method of this example, and experiment was conducted using the Western Blot method of Example 7. The results of Western Blot experiment indicated that the expression of PARP-1 spliceosome in the polypeptide CPP-1 and ATO combined action group was higher than those of other groups (as shown in FIG. 39), which suggested that the apoptosis rate of that group was the highest and the results were consistent with the results of Annexin V-PE/7-AAD flow cytometry. The results demonstrated that polypeptide CPP-1 can accelerate ATO-induced tumor apoptosis.

Thereafter, the inventors of this patent continued to conduct research on the effects of singly using polypeptide CPP-JWA-6 and using polypeptide CPP-JWA-6 in combination with ATO for treatment of A375 cells and SGC7901 cells on the apoptosis thereof. The sequence of polypeptide CPP-JWA-6 was SEQ ID NO. 14, i.e. 6-aminocaproic acid-(R)₉-FPGSDRF, and the amino acid S was phosphorylated.

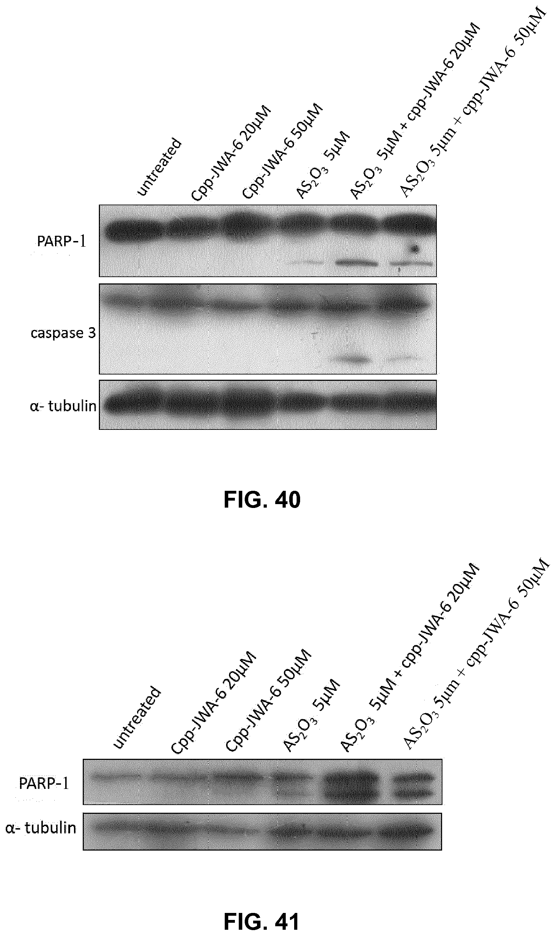

Polypeptide CPP-JWA-6 at a dose of 20 µM, 50 µM was applied individually and applied in combination with ATO (5 µM) on A375 cells (as shown in FIG. 40) and SGC7901 cells (as shown in FIG. 41) for 24 hrs before protein was extracted, and the Western Blot method of Example 7 was used to examine the changes in apoptosis-related molecules.

The results indicated that polypeptide CPP-JWA-6 at a dose not exceeding 50 µM will not cause any changes in the expression of PARP-1; in comparison with the single application of ATO, caspase-3 spliceosome and PARP-1 spliceosome were significantly increased after the combined action of CPP-JWA-6 and ATO, which indicated that polypeptide CPP-JWA-6 can also accelerate ATO-induced tumor apoptosis.

### Example 10 Selecting functional polypeptides with polypeptide JWA-6 as the active site

The objective of this example was to select functional polypeptides from multiple combinations of sequences based on polypeptide JWA-6.

The mice with transplanted tumors in this example were the same as Example 2, and the experimental method used therein was also the same as Example 2. The various polypeptides were administered by intratumoral injection at a dose of 10 mg/kg, and a non-pep injection group (referred to simply as "non-pep group", the polypeptide sequence used was the same as Example 3) was established.

The sequences of various polypeptides were recorded as shown in Table 4.

**Table 4 Sequences of various combinations of polypeptides**

| Name | Amino Acid Sequence (N-terminus - C-terminus) | Phosphorylation Site |
|---|---|---|
| J1 | FPGSDRF | S phosphorylated |
| J2 | F-FPGSDRF | S phosphorylated |
| J3 | 6-aminocaproic acid-F-FPGSDRF | S phosphorylated |
| J4 | (R)₉-FPGSDRF | S phosphorylated |
| J5 | (R)₉-F-FPGSDRF | S phosphorylated |
| J6 | 6-aminocaproic acid-(R)₉-F-FPGSDRF | S phosphorylated |
| J7 | FPGSDRF-A | S phosphorylated |
| J8 | FPGSDRF-RGD | S phosphorylated |
| J9 | FPGSDRF-(G)₄-RGD | S phosphorylated |
| J10 | FPGSDRF-(G)₁₀-RGD | S phosphorylated |
| J11 | FPGSDRF-A-RGD | S phosphorylated |
| J12 | FPGSDRF-A-(G)₄-RGD | S phosphorylated |
| J13 | FPGSDRF-A-(G)₁₀-RGD | S phosphorylated |
| J14 | F-FPGSDRF-A | S phosphorylated |
| J15 | 6-aminocaproic acid-FPGSDRF-A | S phosphorylated |
| J16 | (R)₉-FPGSDRF-A | S phosphorylated |
| J17 | (R)₉-F-FPGSDRF-A | S phosphorylated |
| J18 | 6-aminocaproic acid-(R)₉-FPGSDRF-A | S phosphorylated |
| J19 | F-FPGSDRF-RGD | S phosphorylated |
| J20 | F-FPGSDRF-(G)₄-RGD | S phosphorylated |
| J21 | F-FPGSDRF-(G)₁₀-RGD | S phosphorylated |
| J22 | 6-aminocaproic acid-F-FPGSDRF-RGD | S phosphorylated |
| J23 | 6-aminocaproic acid-F-FPGSDRF-(G)₄-RGD | S phosphorylated |
| J24 | 6-aminocaproic acid-F-FPGSDRF-(G)₁₀-RGD | S phosphorylated |
| J25 | (R)₉-FPGSDRF-RGD | S phosphorylated |
| J26 | (R)₉-FPGSDRF-(G)₄-RGD | S phosphorylated |
| J27 | (R)₉-FPGSDRF-(G)₁₀-RGD | S phosphorylated |
| J28 | (R)₉-F-FPGSDRF-RGD | S phosphorylated |
| J29 | (R)₉-F-FPGSDRF-(G)₄-RGD | S phosphorylated |
| J30 | (R)₉-F-FPGSDRF-(G)₁₀-RGD | S phosphorylated |
| J31 | 6-aminocaproic acid-(R)₉-FPGSDRF-RGD | S phosphorylated |
| J32 | 6-aminocaproic acid-(R)₉-FPGSDRF-(G)₄-RGD | S phosphorylated |
| J33 | 6-aminocaproic acid-(R)₉-FPGSDRF-(G)₁₀-RGD | S phosphorylated |
| J34 | 6-aminocaproic acid-(R)₉-F-FPGSDRF-RGD | S phosphorylated |
| J35 | 6-aminocaproic acid-(R)₉-F-FPGSDRF-(G)₄-RGD | S phosphorylated |
| J36 | 6-aminocaproic acid-(R)₉-F-FPGSDRF-(G)₁₀-RGD | S phosphorylated |
| J37 | F-FPGSDRF-A-RGD | S phosphorylated |
| J38 | F-FPGSDRF-A-(G)₄-RGD | S phosphorylated |
| J39 | F-FPGSDRF-A-(G)₁₀-RGD | S phosphorylated |
| J40 | 6-aminocaproic acid-FPGSDRF-A-RGD | S phosphorylated |
| J41 | 6-aminocaproic acid-FPGSDRF-A-(G)₄-RGD | S phosphorylated |
| J42 | 6-aminocaproic acid-FPGSDRF-A-(G)io-RGD | S phosphorylated |
| J43 | 6-aminocaproic acid-F-FPGSDRF-A-RGD | S phosphorylated |
| J44 | 6-aminocaproic acid-F-FPGSDRF-A-(G)₄-RGD | S phosphorylated |
| J45 | 6-aminocaproic acid-F-FPGSDRF-A-(G)₁₀-RGD | S phosphorylated |
| J46 | (R)₉-FPGSDRF-A-RGD | S phosphorylated |
| J47 | (R)₉-FPGSDRF-A-(G)₄-RGD | S phosphorylated |
| J48 | (R)₉-FPGSDRF-A-(G)₁₀-RGD | S phosphorylated |
| J49 | (R)₉-F-FPGSDRF-A-RGD | S phosphorylated |
| J50 | (R)₉-F-FPGSDRF-A-(G)₄-RGD | S phosphorylated |
| J51 | (R)₉-F-FPGSDRF-A-(G)₁₀-RGD | S phosphorylated |
| J52 | 6-aminocaproic acid-(R)₉-FPGSDRF-A-RGD | S phosphorylated |
| J53 | 6-aminocaproic acid-(R)₉-FPGSDRF-A-(G)₄-RGD | S phosphorylated |
| J54 | 6-aminocaproic acid-(R)₉-FPGSDRF-A-(G)₁₀-RGD | S phosphorylated |
| J55 | 6-aminocaproic acid-(R)₉-F-FPGSDRF-A-RGD | S phosphorylated |
| J56 | 6-aminocaproic acid-(R)₉-F-FPGSDRF-A-(G)₄-RGD | S phosphorylated |
| J57 | 6-aminocaproic acid-(R)₉-F-FPGSDRF-A-(G)₁₀-RGD | S phosphorylated |

Because of space constraints, the actual experimental data is not listed herein. The experimental data indicated that polypeptides 1-57 have anti-tumor activity.

Unless otherwise indicated, the numerical ranges involved in the invention include the end values.

## Claims

1. An anti-tumor polypeptide, having an amino acid sequence represented by
FPGSDRF;
X-FPGSDRF;
FPGSDRF-Z; or
X-FPGSDRF-Z;
**characterized in that**
S is a phosphorylated serine residue;
X and Z are an amino acid residue or an amino acid sequence, respectively;
X is one selected from the group consisting of F, (R)₉, (R)₉-F, 6-aminocaproic acid, 6-aminocaproic acid-F, 6-aminocaproic acid-(R)₉, and 6-aminocaproic acid-(R)₉-F;
Z is one selected from the group consisting of A, (G)ₙ-RGD, and A-(G)ₙ-RGD; and
n is an integer greater than or equal to 0.

2. The polypeptide of claim 1, **characterized in that** a range of n is between 0 and 10.

3. The polypeptide of claim 2, **characterized in that** an N-terminus of the polypeptide is acetylated and a C-terminus of the polypeptide is amidated.

4. A method for preparing an anti-tumor drug comprising using the anti-tumor polypeptide of any one of claims 1-3.

5. The method of claim 4, **characterized in that** the tumor is melanoma or gastric carcinoma.

## Patentansprüche

1. Antitumor-Polypeptid mit einer Aminosäuresequenz, dargestellt durch
FPGSDRF;
X-FPGSDRF;
FPGSDRF-Z; oder
X-FPGSDRF-Z;
**dadurch gekennzeichnet, dass**
S ein phosphorylierter Serinrest ist;
X und Z ein Aminosäurerest bzw. eine Aminosäuresequenz ist;
X ausgewählt ist aus der Gruppe bestehend aus F, (R)g, (R)₉-F, 6-Aminocapronsäure, 6-Aminocapronsäure-F, 6-Aminocapronsäure- (R)₉, und 6-Aminocapronsäure- (R)₉-F;
Z ausgewählt ist aus der Gruppe bestehend aus A, (G)ₙ-RGD und A- (G)ₙ-RGD; und
n eine ganze Zahl größer oder gleich Null ist.

2. Polypeptid nach Anspruch 1, **dadurch gekennzeichnet, dass** n in einem Bereich zwischen 0 und 10 liegt.

3. Polypeptid nach Anspruch 2, **dadurch gekennzeichnet, dass** ein N-Terminus des Polypeptids acetyliert und ein C-Terminus des Polypeptids amidiert ist.

4. Verfahren zur Herstellung eines Antitumorarzneimittels, umfassend die Verwendung des Antitumorpolypeptids nach einem der Ansprüche 1 bis 3.

5. Verfahren nach Anspruch 4, **dadurch gekennzeichnet, dass** der Tumor ein Melanom oder Magenkarzinom ist.

## Revendications

1. Polypeptide anti-tumoral, ayant une séquence d'acides aminés représentée par
FPGSDRF;
X-FPGSDRF ;
FPGSDRF-Z ; ou
X-FPGSDRF-Z ;
**caractérisé en ce que**
S est un résidu sérine phosphorylé ;
X et Z sont un résidu acide aminé ou une séquence d'acides aminés respectivement ;
X est sélectionné dans le groupe constitué par F, (R)₉, (R)₉-F, l'acide 6-aminocaproïque, l'acide 6-aminocaproïque-F, l'acide 6-aminocaproïque-(R)₉, et l'acide 6-aminocaproïque-(R)₉-F ;
Z est sélectionné dans le groupe constitué par A, (G)ₙ-RGD, et A-(G)n-RGD ; et
n est un nombre entier supérieur ou égal à 0.

2. Polypeptide selon la revendication 1, **caractérisé en ce qu'**une plage de n est comprise entre 0 et10.

3. Polypeptide selon la revendication 2, **caractérisé en ce qu'**une extrémité N-terminale du polypeptide est acétylée et une extrémité C-terminale du polypeptide est amidée.

4. Procédé de préparation d'un médicament anti-tumoral comprenant l'utilisation du polypeptide anti-tumoral selon l'une quelconque des revendications 1 à 3.

5. Procédé selon la revendication 4, **caractérisé en ce que** la tumeur est un mélanome ou un carcinome gastrique.


### SEQUENCE LISTING

<110> Nanjing Medical Univeristy
<120> POLYPEPTIDE HAVING ANTI-TUMOR ACTIVITY AND USE THEREOF
<130> NJYK-00103-NEP
<160> 15
<170> PatentIn version 3.5
<210> 1
   <211> 9
   <212> PRT
   <213> Artificial Sequence
<220>
   <223> Fully synthetic
<220>
   <221> MOD_RES
   <222> (1)..(1)
   <223> Acp
<220>
   <221> MOD_RES
   <222> (5)..(5)
   <223> PHOSPHORYLATION
<400> 1
   Phe Phe Pro Gly Ser Asp Arg Phe Ala
   1               5
<210> 2
   <211> 9
   <212> PRT
   <213> Artificial Sequence
<220>
   <223> Fully synthetic
<220>
   <221> MOD_RES
   <222> (1)..(1)
   <223> Acp
<220>
   <221> MOD_RES
   <222> (5)..(5)
   <223> PHOSPHORYLATION
<400> 2
   Phe Ile His Ala Ser Leu Arg Leu Arg
   1 5
<210> 3
   <211> 8
   <212> PRT
   <213> Artificial Sequence
<220>
   <223> Fully synthetic
<220>
   <221> MOD_RES
   <222> (1)..(1)
   <223> Acp
<220>
   <221> MOD_RES
   <222> (4)..(4)
   <223> PHOSPHORYLATION
<400> 3
   Leu Thr Asp Tyr Ile Ser Lys Val
   1 5
<210> 4
   <211> 4
   <212> PRT
   <213> Artificial Sequence
<220>
   <223> Fullly synthetic
<220>
   <221> MOD_RES
   <222> (1)..(1)
   <223> Xaa is Acp
<220>
   <221> MOD_RES
   <222> (3)..(3)
   <223> PHOSPHORYLATION
<400> 4
   Xaa Gly Ser Asp
   1
<210> 5
   <211> 5
   <212> PRT
   <213> Artificial Sequence
<220>
   <223> Fully synthetic
<220>
   <221> MOD_RES
   <222> (1)..(1)
   <223> Acp
<220>
   <221> MOD_RES
   <222> (3)..(3)
   <223> PHOSPHORYLATION
<400> 5
   Pro Gly Ser Asp Arg
   1               5
<210> 6
   <211> 7
   <212> PRT
   <213> Artificial Sequence
<220>
   <223> Fully synthetic
<220>
   <221> MOD_RES
   <222> (1)..(1)
   <223> Acp
<220>
   <221> MOD_RES
   <222> (4)..(4)
   <223> PHOSPHORYLATION
<400> 6
   Phe Pro Gly Ser Asp Arg Phe
   1               5
<210> 7
   <211> 9
   <212> PRT
   <213> Artificial Sequence
<220>
   <223> Fully synthetic
<220>
   <221> MOD_RES
   <222> (1)..(1)
   <223> Acp
<400> 7
   Phe Phe Pro Gly Ser Asp Arg Phe Ala
   1               5
<210> 8
   <211> 14
   <212> PRT
   <213> Artificial Sequence
<220>
   <223> Fully synthetic
<220>
   <221> MOD_RES
   <222> (1)..(1)
   <223> Acp
<220>
   <221> MOD_RES
   <222> (4)..(4)
   <223> PHOSPHORYLATION
<400> 8
   Phe Pro Gly Ser Asp Arg Phe Gly Gly Gly Gly Arg Gly Asp
   1               5                   10
<210> 9
   <211> 9
   <212> PRT
   <213> Artificial Sequence
<220>
   <223> Fully synthetic
<220>
   <221> MOD_RES
   <222> (1)..(1)
   <223> Acp
<400> 9
   Arg Arg Arg Arg Arg Arg Arg Arg Arg
   1               5
<210> 10
   <211> 9
   <212> PRT
   <213> Artificial Sequence
<220>
   <223> Fully synthetic
<220>
   <221> MOD_RES
   <222> (1)..(1)
   <223> Acp
<400> 10
   Arg Lys Lys Arg Arg Gln Arg Arg Arg
   1               5
<210> 11
   <211> 21
   <212> PRT
   <213> Artificial Sequence
<220>
   <223> Fully synthetic
<220>
   <221> MOD_RES
   <222> (1)..(1)
   <223> Acp
<400> 11
   Lys Glu Thr Trp Trp Glu Thr Trp Trp Thr Glu Trp Ser Gln Pro Lys
   1               5                   10                  15
   Lys Lys Arg Lys Val
               20
<210> 12
   <211> 11
   <212> PRT
   <213> Artificial Sequence
<220>
   <223> Fully synthetic
<220>
   <221> MOD_RES
   <222> (1)..(1)
   <223> Acp
<400> 12
   Tyr Gly Arg Lys Lys Arg Arg Gln Arg Arg Arg
   1               5                   10
<210> 13
   <211> 18
   <212> PRT
   <213> Artificial Sequence
<220>
   <223> Fully synthetic
<220>
   <221> MOD_RES
   <222> (1)..(1)
   <223> Acp
<220>
   <221> MOD_RES
   <222> (14)..(14)
   <223> PHOSPHORYLATION
<400> 13
   Arg Arg Arg Arg Arg Arg Arg Arg Arg Phe Phe Pro Gly Ser Asp Arg
   1 5 10 15
   Phe Ala
<210> 14
   <211> 16
   <212> PRT
   <213> Artificial Sequence
<220>
   <223> Fully synthetic
<220>
   <221> MOD_RES
   <222> (1)..(1)
   <223> Acp
<220>
   <221> MOD_RES
   <222> (13)..(13)
   <223> PHOSPHORYLATION
<400> 14
   Arg Arg Arg Arg Arg Arg Arg Arg Arg Phe Pro Gly Ser Asp Arg Phe
   1 5 10 15
<210> 15
   <211> 6
   <212> PRT
   <213> Artificial Sequence
<220>
   <223> Fully synthetic
<220>
   <221> MOD_RES
   <222> (1)..(1)
   <223> ACETYLATION, Acp
<220>
   <221> MOD_RES
   <222> (5)..(5)
   <223> AMIDATION
<220>
   <221> MOD_RES
   <222> (6)..(6)
   <223> AMIDATION
<400> 15
   Glu Glu Met Gln Arg Arg
   1 5